Europäisches Patentamt

European Patent Office

Office européen des brevets

(19)

(11) Numéro de publication : **0 043 620**
B1

(12) **FASCICULE DE BREVET EUROPÉEN**

(45) Date de publication du fascicule du brevet :
30.01.85

(21) Numéro de dépôt : 81200724.3

(22) Date de dépôt : 26.06.81

(51) Int. Cl.⁴ : **C 07 C 59/01**, C 07 C 51/09,
C 07 C 69/675,
C 07 C 67/333// C08L67/04,
C08J11/00, C12P7/62,
C12P7/42

(54) **Procédé pour la fabrication de l'acide bêta-hydroxybutyrique.**

(30) Priorité : 03.07.80 FR 8014889

(43) Date de publication de la demande :
13.01.82 Bulletin 82/02

(45) Mention de la délivrance du brevet :
30.01.85 Bulletin 85/05

(84) Etats contractants désignés :
AT BE CH DE FR GB IT LI NL SE

(56) Documents cités :
FR-A- 2 369 343
US-A- 3 044 942
MACROMOLECULES, vol. 9, no. 5, septembre-octobre 1976 S. AKITA et al. "Solution properties of
poly(d-beta-hydroxy-butyrate)", pages 774-780
CHEMICAL ABSTRACTS, vol. 83, no. 17, 27 octobre
1975 page 171, abrégé 143344q Columbus, Ohio, USA
HAUTTECOEUR, B et al. "Mild chemical depolymerization of beta-hydroxybutyric lipid (PHB) of Bacillus
megaterium. Action of boron trifluoride-methanol
mixture on polymers (and oligomers) of D-(-)-3-
hydroxybutyric acid"
Hauttecœur et al, C.R. Acad Sci. Paris, 280 (1975)
2899-2902

(73) Titulaire : SOLVAY & Cie (Société Anonyme)
Rue du Prince Albert, 33
B-1050 Bruxelles (BE)

(72) Inventeur : Vanlautem, Noel
Rue des Drapiers, 17
B-1300 Wavre (BE)
Inventeur : Gilain, Jacques
Avenue de la Ramée 10
B-1180 Bruxelles (BE)

Jouve, 18, rue St-Denis, 75001 Paris, France

# 0 043 620

## Description

La présente invention concerne un procédé pour la fabrication de l'acide β-hydroxybutyrique et de ses oligocondensats à partir de ses polycondensats obtenus par biosynthèse.

De nombreux microorganismes sont capables de synthétiser des polycondensats de l'acide β-hydroxybutyrique (poly-β-hydroxybutyrates) et diverses techniques, telles que l'extraction, sont connues pour séparer les poly-β-hydroxybutyrates des biomasses.

Comme l'acide β-hydroxybutyrique est un produit intermédiaire très intéressant pour l'industrie chimique fine, diverses tentatives ont été réalisées en vue de le synthétiser au départ de ses polycondensats obtenus par biosynthèse. Cependant, tous les procédés envisagés jusqu'à maintenant à cet effet présentent l'inconvénient grave de conduire non pas à l'acide lui-même mais à des dérivés. Ces procédés connus nécessitent donc la conversion ultérieure de ces dérivés en l'acide, ce qui les rend trop compliqués et trop onéreux pour pouvoir être utilisés industriellement.

Ainsi, une première de ces méthodes connues consiste à saponifier les poly-β-hydroxybutyrates au moyen d'une solution alcaline de manière à former des sels alcalins de l'acide β-hydroxybutyrique. Cette méthode comporte l'inconvénient supplémentaire d'entraîner la formation de sous-produits non désirés tels que le sel de l'acide crotonique en quantités importantes. Une deuxième méthode déjà proposée consiste à traiter les poly-β-hydroxybutyrates au moyen d'hydrazine anhydre ce qui permet l'obtention de cristaux d'hydrazide de l'acide D(−)-β-hydroxybutyrique, lequel ne peut être transformé en acide qu'à l'intervention de quantités stœchiométriques d'acides tels que l'acide sulfurique ou l'acide chlorhydrique, les rendements observés étant par ailleurs très faibles. Selon une troisième méthode connue, on opère par méthanolyse et on obtient des oligocondensats sous la forme d'esters méthyliques ainsi que l'ester méthylique de l'acide. Un exemple d'un tel procédé de méthanolyse est décrit dans le document de B. Hauttecœur et al. publié dans C.R. Acad. Sc. Paris, t 280 (30 juin 1975) (25) pages 2899 à 2902. Selon ce procédé, on réalise la dépolymérisation partielle du poly-β-hydroxybutyrate en ses oligomères sous forme d'esters méthyliques, à l'intervention d'un mélange constitué de $BF_3$ et de méthanol. Un autre exemple de cette technique de méthanolyse est décrit par S. Akita et al. dans MACROMOLECULES, Volume 9, numéro 5, 1976, 774-780 dans le cadre de la détermination de certaines propriétés du poly-(D-β-hydroxybutyrate) en solution diluée. Ce document divulgue notamment un procédé d'obtention d'oligomères dérivés du poly-(D-β-hydroxybutyrate) sous forme d'esters méthyliques, par addition, à du poly(D-β-hydroxybutyrate), extrait d'une biomasse dissoute dans le dichlorométhane, d'une solution de méthanol contenant de l'acide paratoluènesulfonique.

La présente invention vise à fournir un procédé qui conduise directement à l'acide β-hydroxybutyrique.

Elle concerne à cet effet un procédé pour la fabrication de l'acide β-hydroxybutyrique à partir de ses polycondensats selon lequel on soumet les polycondensats à une hydrolyse dans un mélange réactionnel liquide homogène contenant un solvant, des polycondensats, un catalyseur acide et de l'eau.

Les poly-β-hydroxybutyrates, également appelés acides poly-β-hydroxybutyriques, mis en œuvre selon l'invention peuvent être des produits bruts ou des produits purs obtenus à partir d'une biomasse par tout moyen de séparation connu. L'origine de la biomasse est sans importance pour le procédé selon l'invention. La biomasse peut donc provenir de divers microorganismes et notamment de bactéries comme il a été décrit dans Angewandte Chemie, 1962, 74ème année, N° 10, pages 342 à 346 par Schlegel et Gottschalk.

De préférence, on utilise comme matière première des poly-β-hydroxybutyrates séparés de la biomasse par extraction au moyen de solvants qui sont utilisables dans le procédé selon l'invention et décrits ci-après. Cela permet de réaliser le procédé selon l'invention directement après l'extraction, dans le même solvant, sans discontinuité entre les opérations.

Les poly-β-hydroxybutyrates mis en œuvre ont en général une masse moléculaire moyenne en poids ($\overline{M}_w$) supérieure à 100.000. Habituellement, cette masse moléculaire est comprise entre 500.000 et 3.000.000, et le plus souvent entre 700.000 et 1.500.000. Elle peut varier en fonction de la provenance du poly-β-hydroxybutyrate mis en œuvre.

Dans le procédé selon l'invention, on met en œuvre un mélange réactionnel dans lequel le solvant, les poly-β-hydroxybutyrates et l'eau ne constituent qu'une phase liquide homogène. Lorsque le catalyseur acide est liquide, il est choisi de préférence de manière à se trouver sous forme totalement dissoute dans cette phase liquide. On opère donc de préférence en l'absence d'une seconde phase liquide ou d'une phase solide comportant des poly-β-hydroxybutyrates non dissous.

Les solvants mis en œuvre selon l'invention peuvent être choisis parmi tous les solvants organiques capables de solubiliser les poly-β-hydroxybutyrates tout en étant inertes chimiquement vis-à-vis de ceux-ci, de l'eau et du catalyseur acide utilisé. Les solvants peuvent être constitués d'un seul composé, pur ou de qualité technique, ou d'un mélange de plusieurs composés.

De préférence, on choisit les solvants parmi ceux utilisables pour l'extraction des poly-β-hydroxybutyrates des biomasses et en particulier parmi les solvants halogénés, substitués ou, de préférence, non substitués. Parmi ceux-ci, les chloroéthanes et chloropropanes décrits dans le brevet français 7 901 862 déposé le 22-1-1979 au nom de SOLVAY & Cie ainsi que le chlorure de méthylène et le chloroforme

2

conviennent bien. On a obtenu de bons résultats avec le chlorure de méthylène, le chloroforme, le 1,2-dichloréthane, le 1,1,2-trichloroéthane, le 1,1,2,2-tétrachloroéthane et le 1,2,3-trichloropropane. Les meilleurs résultats ont été obtenus avec le chloroforme et tout particulièrement avec le 1,2-dichloréthane.

Les quantités de solvant par rapport aux quantités de poly-β-hydroxybutyrates à mettre en œuvre peuvent varier entre de larges limites et ne sont pas critiques. La vitesse d'hydrolyse étant toutefois fonction de la concentration en polymère, on préfère travailler avec des concentrations aussi élevées que possible.

Les poly-β-hydroxybutyrates étant d'autant plus solubles dans les solvants que leur masse moléculaire est faible, il est avantageux de concentrer le mélange réactionnel au fur et à mesure que l'hydrolyse progresse. On peut, à cet effet, avantageusement distiller progressivement le solvant de manière à concentrer de plus en plus le mélange réactionnel.

Les catalyseurs acides, que l'on met en œuvre selon l'invention, peuvent être choisis parmi tous les acides de Brönsted et de Lewis. On préfère habituellement utiliser les acides de Brönsted dont le pK$_A$ est inférieur à 4 et, encore de préférence, à 2,5. Parmi ceux-ci, on préfère employer l'acide sulfurique, l'acide chlorhydrique, l'acide phosphorique et leurs dérivés ainsi que les acides sulfoniques. Les résines sulfonées conviennent également bien. Les catalyseurs ayant donné les meilleurs résultats sont les acides alkyl-, aryl-, alkylaryl-, et arylalkylsulfoniques ainsi que l'acide sulfurique. Tout particulièrement préférés sont l'acide sulfurique et l'acide para-toluène sulfonique.

La quantité de catalyseur acide à mettre en œuvre dans le mélange réactionnel n'est pas critique en elle-même. Les concentrations relatives en catalyseur par rapport aux unités monomériques présentes dans les poly-β-hydroxybutyrates mis en œuvre peuvent atteindre des valeurs de 100 % molaires. Ce rapport est toutefois habituellement compris entre 0,1 et 40 % et de préférence entre 0,5 à 10 %.

L'eau peut être introduite dans le milieu réactionnel à tout moment, avant ou pendant l'hydrolyse. Ainsi, le procédé selon l'invention permet de travailler avec des poly-β-hydroxybutyrates bruts ne se trouvant pas sous forme anhydre et avec des solvants et des catalyseurs de qualité technique non anhydres.

La quantité d'eau à mettre en œuvre dans le mélange réactionnel est choisie de préférence de manière qu'à aucun moment, il n'y ait hétérogénéité du mélange réactionnel liquide par apparition de deux phases liquides distinctes, à savoir une phase organique et une phase aqueuse. Il en résulte que, dans ce cas, la quantité d'eau mise en œuvre est choisie en fonction de la nature du solvant, de la température, de la pression et de la masse moléculaire du poly-β-hydroxybutyrate au moment considéré. L'eau étant généralement peu miscible dans les solvants utilisés de préférence selon l'invention, son addition se fait donc avantageusement en plusieurs stades ou en continu tout au long de l'hydrolyse en prenant soin de ne pas dépasser la limite de solubilité de l'eau dans le mélange réactionnel.

Par ailleurs, il a été constaté que la solubilité de l'eau dans le mélange réactionnel augmente au fur et à mesure que l'hydrolyse progresse et que la masse moléculaire des poly-β-hydroxybutyrates diminue. En outre, lorsque l'hydrolyse a progressé de manière telle que le degré de polymérisation a atteint une valeur allant de 2 à 10 environ et que l'on se trouve donc en présence d'oligocondensats, la solubilité de ceux-ci dans l'eau est suffisante pour que l'on puisse poursuivre l'hydrolyse dans une phase liquide aqueuse. A cet effet, on peut éliminer le solvant par une simple distillation et ajouter éventuellement de l'eau. On peut ainsi poursuivre l'hydrolyse en phase liquide aqueuse homogène et obtenir l'acide β-hydroxybutyrique avec des rendements élevés, sans formation de sous-produits en quantités élevées.

Outre le solvant, les poly-β-hydroxybutyrates, l'eau et le catalyseur acide, le mélange réactionnel peut contenir d'autres constituants divers. Parmi ceux-ci, on peut citer les agents de blanchiment tels que les composés peroxydés du type peroxyde d'hydrogène permettant d'obtenir des produits dépolymérisés ayant une blancheur accrue. Ceci se révèle être particulièrement intéressant si l'on opère au départ de poly-β-hydroxybutyrates non purifiés au préalable. La concentration de ces agents de blanchiment doit être ajustée de façon à ne pas provoquer d'altérations chimiques des produits.

Le procédé selon l'invention peut être réalisé à basse température mais habituellement, on opère à des températures supérieures à 25 °C. En général, pour des raisons de commodité, on n'opère pas à des températures supérieures à 150 °C. De préférence, on travaille à des températures situées entre 50 et 125 °C. Par ailleurs, il est particulièrement avantageux d'opérer à la température de reflux du milieu à la pression considérée de manière à pouvoir éliminer progressivement le solvant par distillation comme on l'a expliqué ci-dessus.

La pression appliquée au cours du procédé n'est pas critique en elle-même et est généralement inférieure à 10 bar. De préférence, on travaille à des pressions comprises entre 0,05 et 5 bar. Tout particulièrement, on préfère travailler à la pression atmosphérique.

En ce qui concerne la durée de l'hydrolyse, il va de soi que celle-ci est choisie en fonction du produit que l'on souhaite obtenir. Plus particulièrement, dans le cas où on souhaite obtenir des oligocondensats, cette durée doit être déterminée, cas par cas, en fonction du degré de polymérisation des oligocondensats que l'on souhaite obtenir. Ainsi, le procédé selon l'invention permet d'obtenir facilement des oligocondensats dont la masse moléculaire moyenne en poids est inférieure à 50.000, et plus particulièrement inférieure à 10.000.

Le procédé selon l'invention peut être réalisé en continu ou en discontinu dans n'importe quelle installation permettant de réunir les conditions opératoires décrites ci-dessus.

L'acide β-hydroxybutyrique ou les oligocondensats obtenus par le procédé selon l'invention peuvent être séparés du milieu réactionnel par n'importe quel moyen connu. Habituellement cette opération est effectuée par une ou plusieurs distillations et filtrations suivies ou non de lavages par de l'eau ou des solvants organiques.

Les oligocondensats de l'acide β-hydroxybutyrique peuvent être utilisés pour leurs fonctions hydroxyles ou carboxyles et notamment comme réserve énergétique dans la nourriture animale.

Les exemples suivants servent à illustrer l'invention.

### Exemple 1 (comparatif)

Dans un ballon de 250 cm³ équipé d'un agitateur, d'un thermomètre, d'une ampoule et d'un condenseur à eau, on introduit 4,3 g d'un poly-β-hydroxybutyrate de masse moléculaire moyenne en poids ($\overline{M}_w$) égale à 963.000, dispersés dans 100 cm³ d'eau déminéralisée. On ajoute 2,9 cm³ d'acide sulfurique 35 N au milieu et on porte à ébullition sous agitation mécanique pendant 6 heures. Le milieu réactionnel est recueilli par filtration sur filtre G4 et lavé abondamment à l'eau déminéralisée. Après séchage sous 2,5 kPa à 50 °C jusqu'à poids constant, on récupère 4,22 g d'un poly-β-hydroxybutyrate de masse moléculaire moyenne $\overline{M}_w$ égale à 233.000.

### Exemple 2

Dans un ballon de 500 cm³, muni d'un agitateur, d'un thermomètre, d'une ampoule et d'un condenseur à eau, on introduit 4,3 g d'un poly-β-hydroxybutyrate de masse moléculaire moyenne en poids ($\overline{M}_w$) égale à $1,3.10^6$ que l'on dissout dans 250 cm³ de 1,2-dichloréthane par chauffage du solvant à reflux. On ajoute, après dissolution totale du polymère, 0,43 g d'acide para-toluène sulfonique monohydraté et 4,5 g d'eau. Le mélange, devenu hétérogène par apparition de deux phases liquides est encore chauffé à reflux pendant 6 heures et des prélèvements de 5 cm³ de mélange réactionnel sont effectués au cours de cette opération. On obtient dans chaque cas un poly-β-hydroxybutyrate dont le poids moléculaire est indiqué au Tableau I ci-dessous.

### Tableau I

| Prélèvement | Masse moléculaire moyenne en nombre ($\overline{M}_n$) | Masse moléculaire moyenne en poids ($\overline{M}_w$) |
|---|---|---|
| après 2 h. | 7.800 | 16.000 |
| après 3 h. | 4.300 | 10.000 |
| après 4 h. | 4.300 | 10.000 |
| après 5 h. | 4.300 | 10.000 |

### Exemple 3

On opère comme dans l'exemple 2 mais au lieu de travailler en milieu hétérogène, on opère en milieu homogène c'est-à-dire que l'on utilise un milieu ne contenant que 0,81 g d'eau ce qui à la température d'ébullition (environ 80 °C) constitue une quantité parfaitement miscible au 1,2-dichloréthane. Les résultats observés sont rassemblés au Tableau II ci-après.

### Tableau II

| Prélèvement | Masse moléculaire moyenne en nombre ($\overline{M}_n$) | Masse moléculaire moyenne en poids ($\overline{M}_w$) |
|---|---|---|
| après 2 h. | 1.890 | 4.000 |
| après 3 h. | 1.320 | 2.800 |
| après 4 h. | 1.140 | 2.300 |
| après 5 h. | 970 | 2.000 |

4

La comparaison des exemples 1, 2 et 3 montre que l'hydrolyse des poly-β-hydroxybutyrates en milieu hétérogène aqueux (exemple 1) est insignifiante. La comparaison des exemples 2 et 3 montre que cette hydrolyse en milieu mixte solvant organique-eau hétérogène (exemple 2) est plus efficace mais néanmoins plus lente que l'hydrolyse effectuée en milieu solvant organique homogène (exemple 3). De plus, elle permet d'atteindre les masses moléculaires les plus faibles.

## Exemple 4

Dans le réacteur de l'exemple 1, on introduit 125 cm³ d'une solution à 40 g/l de poly-β-hydroxybutyrate dans le 1,2-dichloréthane provenant de l'extraction. La masse moléculaire moyenne en poids ($\overline{M}_w$) est égale à $1,33.10^6$. On y ajoute 1,06 cm³ d'$H_2SO_4$ 35 N. Ce mélange est porté à ébullition à pression atmosphérique. La solution est concentrée par distillation du solvant et addition en continu de 635 cm³ de la solution d'extraction de manière à maintenir le volume réactionnel à 125 cm³. Après 3 h 30, on obtient un poly-β-hydroxybutyrate de masse moléculaire moyenne en nombre ($\overline{M}_n$) égale à 4.000 et dont la concentration est de 2,83 mole/l.

On place ensuite le condenseur à eau verticalement sur le ballon de façon à pouvoir chauffer la solution à reflux sans élimination de solvant. On ajoute ensuite à la solution portée à la température de reflux en moins de 1 minute 3,2 g d'eau. Après 4,0 heures on ajoute de nouveau 1,6 g d'eau et après 7,7 h. d'ébullition à nouveau 1,6 g d'eau. Après 9 heures, on a obtenu, après épuration et séchage à poids constant, des oligocondensats de masse moléculaire moyenne en nombre ($\overline{M}_n$) égale à 650. Ces produits sont de couleur brunâtre.

## Exemple 5

On opère dans les mêmes conditions qu'à l'exemple 4 mais au lieu d'ajouter de l'eau on ajoute les mêmes quantités d'une solution à 30 % de peroxyde d'hydrogène dans l'eau.

La masse moléculaire moyenne en nombre ($\overline{M}_n$) des oligocondensats obtenus est sensiblement la même que celle obtenue à l'exemple 4 mais les oligomères se présentent maintenant sous forme d'une poudre blanche.

Les exemples 4 et 5 montrent que l'on peut effectuer l'hydrolyse de poly-β-hydroxybutyrates bruts, issus directement de la biomasse, à l'aide d'une catalyse acide en milieu homogène solvant organique-eau au moyen d'une solution qui a été concentrée et dépolymérisée partiellement (exemples 4 et 5) et qu'il est possible en utilisant une solution aqueuse d'un composé peroxydé d'obtenir des oligomères parfaitement blancs (exemple 5).

## Exemple 6

Dans un ballon de 500 cm³ d'un agitateur mécanique, d'un thermomètre, d'une ampoule et d'un condenseur à eau permettant de distiller la phase liquide, on introduit, progressivement une solution dans le 1,2-dichloréthane à 40 g/l d'un poly-β-hydroxybutyrate de masse moléculaire moyenne en poids ($\overline{M}_w$) égale à $1,33.10^6$ ainsi que 7,0 g d'acide p. toluènesulfonique et on distille en continu le solvant de façon à obtenir après 4 heures 250 cm³ d'une solution concentrée contenant 256 g/l de poly-β-hydroxybutyrate de masse moléculaire moyenne en nombre ($\overline{M}_n$) égale à 9.200.

Cette solution est ensuite chauffée à reflux dans le même appareil modifié de façon que le condenseur à eau soit monté à la verticale et puisse fonctionner comme colonne de reflux. Lorsque la solution atteint la température de reflux, on ajoute en une fois 6,7 cm³ d'eau et après 4 heures de fonctionnement on ajoute 3,4 cm³ supplémentaires d'eau. La réaction est arrêtée après 6,5 heures et les oligocondensats obtenus ont une masse moléculaire moyenne en nombre ($\overline{M}_n$) égale à 270.

L'appareillage est ensuite de nouveau modifié de façon à pouvoir, comme au départ, fonctionner comme appareil de distillation et le 1,2-dichloréthane est éliminé en continu par distillation en 2,5 heures tandis que l'on ajoute progressivement de l'eau à l'aide de l'ampoule. On chauffe encore pendant 4 heures la solution d'eau à reflux et l'on obtient finalement de l'acide β-hydroxybutyrique lequel est isolé par distillation sous pression réduite, inférieure à 15 Pa, après neutralisation de l'acide p. toluènesulfonique par du NaOH 2N. Le rendement molaire en acide distillé, par rapport au polymère de départ est de 62 %. La formation d'acide crotonique n'a jamais été observée au cours des différentes étapes réactionnelles.

**Revendications**

1. Procédé pour la fabrication de l'acide β-hydroxybutyrique à partir de ses polycondensats caractérisé en ce que l'on soumet les polycondensats à une hydrolyse dans un mélange réactionnel liquide homogène contenant un solvant, des polycondensats, un catalyseur acide et de l'eau.

2. Procédé selon la revendication 1 caractérisé en ce que la phase homogène est une phase organique.

3. Procédé selon les revendications 1 ou 2 caractérisé en ce que l'on choisit comme solvant un solvant halogéné.

4. Procédé selon la revendication 3 caractérisé en ce que l'on choisit le solvant parmi les chloropropanes, les chloroéthanes, le chlorure de méthylène et le chloroforme.

5. Procédé selon les revendications 1 à 4 caractérisé en ce que l'on utilise un catalyseur acide choisi parmi l'acide sulfurique et les acides alkyl-, aryl-, alkylaryl- ou arylalkylsulfoniques.

6. Procédé selon l'une quelconque des revendications 1 à 5 caractérisé en ce que l'on concentre le mélange réactionnel au fur et à mesure de l'hydrolyse par distillation du solvant.

7. Procédé selon l'une quelconque des revendications 1 à 6 caractérisé en ce que l'on élimine le solvant lorsque l'on a obtenu des oligocondensats solubles dans l'eau et on poursuit l'hydrolyse en phase liquide aqueuse.

8. Procédé selon l'une quelconque des revendications 1 à 7 caractérisé en ce que le mélange réactionnel est maintenu à une température comprise entre 50 et 125 °C.

9. Procédé selon l'une quelconque des revendications 1 à 8 caractérisé en ce que l'on ajoute un composé peroxydé au mélange réactionnel.

## Claims

1. Process for the manufacture of β-hydroxybutyric acid from its polycondensates, characterised in that the polycondensates are subjected to hydrolysis in a homogeneous liquid reaction mixture containing a solvent, polycondensates, an acid catalyst and water.

2. Process according to Claim 1, characterised in that the homogeneous phase is an organic phase.

3. Process according to Claims 1 or 2, characterised in that a halogenated solvent is chosen as a solvent.

4. Process according to Claim 3, characterised in that the solvent is chosen from chloro propanes, chloroethanes, methylene chloride and chloroform.

5. Process according to Claims 1 to 4, characterised in that an acid catalyst chosen from sulphuric acid and alkyl-, aryl-, alkylaryl- or arylalkylsulphonic acids is employed.

6. Process according to any one of Claims 1 to 5, characterised in that the reaction mixture is concentrated in step with the hydrolysis by distillation of the solvent.

7. Process according to any one of Claims 1 to 6, characterised in that the solvent is removed when watersoluble oligocondensates have been produced and hydrolysis continues in an aqueous liquid phase.

8. Process according to any one of Claims 1 to 7, characterised in that the reaction mixture is maintained at a temperature of between 50 and 125 °C.

9. Process according to any one of Claims 1 to 8, characterised in that a peroxide compound is added to the reaction mixture.

## Ansprüche

1. Verfahren zur Herstellung von β-Hydroxybuttersäure ausgehend von deren Polykondensaten dadurch gekennzeichnet, daß man die Polykondensate einer Hydrolyse unterwirft in einer flüssigen, homogenen Reaktionsmischung enthaltend ein Lösungsmittel, die Polykondensate, einen Säurekatalysator und Wasser.

2. Verfahren nach Anspruch 1, dadurch gekennzeichnet, daß die homogene Phase eine organische Phase ist.

3. Verfahren nach Anspruch 1 oder 2, dadurch gekennzeichnet, daß man als Lösungsmittel ein halogeniertes Lösungsmittel auswählt.

4. Verfahren nach Anspruch 3, dadurch gekennzeichnet, daß man das Lösungsmittel auswählt unter den Chlorpropanen, Chlorethanen, Methylenchlorid und Chloroform.

5. Verfahren nach einem der Ansprüche 1 bis 4, dadurch gekennzeichnet, daß man einen Säurekatalysator verwendet, der ausgewählt ist unter Schwefelsäure und den Alkyl-, Aryl-, Alkylaryl- oder Arylalkylsulfonsäuren.

6. Verfahren nach einem der Ansprüche 1 bis 5, dadurch gekennzeichnet, daß man die Reaktionsmischung durch Abdestillation des Lösungsmittels im Ausmaß der Hydrolyse konzentriert.

7. Verfahren nach einem der Ansprüche 1 bis 6, dadurch gekennzeichnet, daß man das Lösungsmittel eliminiert wenn man in Wasser lösliche Oligokondensate erhalten hat und die Hydrolyse in flüssiger wässriger Phase fortsetzt.

8. Verfahren nach einem der Ansprüche 1 bis 7, dadurch gekennzeichnet, daß die Reaktionsmischung bei einer Temperatur zwischen 50 und 125 °C gehalten wird.

9. Verfahren nach einem der Ansprüche 1 bis 8, dadurch gekennzeichnet, daß man der Reaktionsmischung eine Peroxydverbindung zusetzt.